# EUROPEAN PATENT APPLICATION

(11) **EP 1 264 600 A1**
(43) Date of publication of application: **11.12.2002**
(21) Application number: 01908150.4
(22) Date of filing: 28.02.2001
(51) Int. Cl.: A61K 39/39, A61K 31/375, A61K 31/665, A61K 47/22, A61K 47/24, A61P 37/04

(54) **IMMUNE ENHANCEMENT COMPOSITIONS AND USE THEREOF**

(30) Priority: 29.02.2000 JP 2000055019; 29.11.2000 US 253744 P
(71) Applicant: SHOWA DENKO K.K., Tokyo 105-8518 (JP)
(72) Inventor: ITO, Shinobu, Musashino-shi, Tokyo 180-0005 (JP); OGATA, Eiji, c/o Showa Denko K.K., Kawasaki-shi, Kanagawa 210-0867 (JP)
(74) Representative: Strehl Schübel-Hopf & Partner
(86) International application number: JP0101512
(87) International publication number: WO01064245

(57) **Abstract**

Immune enhancement compositions for vaccines for virus, bacteria and/or infectious pathogens which contain stable activity-type antioxidant provitamins; a method of enhancing the immunity of vaccines for virus, bacteria and/or infectious pathogens with use of these compositions; and use of the above composition. As the stable activity-type antioxidant provitamins, at least one compound selected from among L-ascorbic acid derivatives and α-tocopheryl phosphates is used.

## Description

### CROSS REFERENCE TO RELATED APPLICATIONS

This application is based on the provisions of 35 U.S.C. Article 111(a) with claiming the benefit of filing dates of U.S. provisional application Serial No. 60/253,744 filed on November 29, 2000 under the provisions of 35 U.S.C. 111(b), pursuant to 35 U.S.C. Article 119(e)(1).

### TECHNICAL FIELD

The present invention relates to a composition for enhancing the immunological effects of a vaccine for viruses, bacteria and/or infectious disease pathogens, containing at least one compound selected from L-ascorbic acid derivatives and α-tocopheryl phosphates, which are stable activity-type antioxidant provitamins (hereinafter referred to as "stable activity-type provitamin") as an active ingredient; a method for enhancing the immunological effects of a vaccine for viruses, bacteria and/or infectious disease pathogens by using the composition; and a use of the composition.

### BACKGROUND OF THE INVENTION

By virtue of the development of a vaccine or an antibiotic, human and animals under feeding, such as cattle, pet and bleeding marine animal, have been prevented from infectious diseases via various viruses or bacteria. In other words, antibiotics specific for bacterial infectious diseases have already succeeded in greatly mitigating the fear for bacterial diseases.

However, antibiotics or chemotherapics effective for viral infectious diseases are small in the number and the predominating prophylaxis is vaccine inoculation represented by poliomyelitis and vaccination.

The vaccine in general includes an "inactivated vaccine" by the inactivation of a pathogenesis virus and a "live vaccine" by the attenuation thereof. Examples of the vaccine practically used for human at present include vaccines of poliomyelitis, measles, rubella, mumps, influenza, Japanese encephalitis, varicella, icterus and hepatitis B. Among these, vaccines of influenza, Japanese encephalitis and hepatitis B belong to the "inactivated vaccine" and others belong to the "live vaccine".

As compared with the inactivated vaccine, the live vaccine is generally advantageous in that the immunity acquisition is close to that by the natural infection and the immunological effects is high. However, the live vaccine is said to have problems such that the toxicity may recover or the quality is unstable. On the other hand, the inactivated vaccine lower in the toxicity than the live vaccine is not completely free of toxicity, and aggressive studies are being made with an attempt to develop more safe and effective component vaccines using genetic engineering means. However, these are also pointed out to have some problems in the practical use.

As described above, many vaccines are being used in practice, however, their phylactic effect is not sufficiently high and various methods for enhancing the immunological effects of vaccine are being studied. For example, use of an adjuvant is one of means for enhancing the effect of vaccine and a long known synthetic Freund's adjuvant is also attempted but this adjuvant disadvantageously causes a strong adverse reaction, that is, has a problem in the safety.

In general, when a vaccine is inoculated to an organism, the immunological effect thereof can be known by measuring the level of the specific antibody in the blood (circulating antibody).

The circulating antibody level can be measured by using hemagglutination inhibition (HI) reaction when the antigen has a hemagglutination activity such as in the case of influenza virus. Specifically, 2-fold step dilution series of a target virus solution are prepared first, a constant amount of erythrocytes are added to each dilution and after the passing of a predetermined time, the agglutination images are examined. The HA unit (HAU) is the reciprocal of the highest dilution number in a test tube showing agglutination positive. Next, 2-fold step dilution series of a serum whose antibody value is to be measured are prepared, the same amount of a virus solution diluted to 4 HAU are added to each of the dilution series and the mixed solution is allowed to stand for a predetermined time. A constant amount of erythrocytes is added to each of these solutions and after the passing of a predetermined time, the agglutination images are examined. The antibody value of the serum (HI unit, HIU) is the reciprocal of the highest dilution number in a test tube in which the agglutination of the erythrocytes is completely inhibited.

When the antigen does not have a hemagglutination activity, the amount of a specific antibody in the blood can be measured by using a passive hemagglutination (PHA) and the like. This method uses erythrocytes to which an antigen has been bound by tannic acid or chromium chloride, etc. and the antibody value of an antiserum is determined therewith as HA unit.

As described above, the live vaccine has a fear of occurrence of mutation of the attenuated toxicity strain *in vivo* into a strongly toxic strain and the inactivated vaccine has a latent danger in essence such that the antigenicity is distorted during the inactivation operation and this gives rise to an unexpected adverse reaction.

Apart from this, group administration of a vaccine is being performed at present for the low ages and therefore, in order to prevent the adverse reaction ascribable to the dispersion of the sensitivity in the host side, a safer and more effective vaccine is being demanded.

Furthermore, among various viruses, in the case of a virus which causes infection and proliferates on the surface of respiratory tract, such as influenza virus, unless the local secretory antibody (IgA antibody) of the respiratory tract is present in a sufficiently high concentration, the virus infection cannot be always prevented even if the antibody concentration in blood is high. At present, subcutaneous inoculation of an inactivated vaccine is employed for the prophylaxis of influenza. However, the IgA antibody will not be increased, although this method is effective for increasing the antibody in blood. Accordingly, for preventing the infection not only by the influenza but also by viruses which cause infection and proliferate on the surface of respiratory tract or on the mucosa of digestive tract, the matter of importance is to increase the IgA antibody in addition to the antibody in blood.

### DISCLOSURE OF THE INVENTION

In order to solve the above-described problems, the present inventors have made extensive investigations on a method for enhancing the immunological effects of a vaccine based on the estimation that when the immunological effects of a vaccine is enhanced, the dose can be reduced and in turn, the frequency in generation of an adverse reaction can be reduced. As a result, it has been found that the above-described problems can be overcome by using a specific antioxidant provitamin in combination with a vaccine. The present invention has been accomplished based on this finding.

More specifically, the present invention relates to the following composition and method for enhancing the immunological effects and use thereof.
1. A composition for enhancing the immunological effects of a vaccine for viruses, bacteria and/or infectious disease pathogens, comprising a stable activity-type antioxidant provitamin.
2. The composition for enhancing the immunological effects as described in the above item 1, wherein the antioxidant provitamin is at least one compound selected from L-ascorbic acid derivatives, α-tocopheryl phosphates and salts thereof.
3. The composition for enhancing the immunological effects as described in the above items 1 or 2, wherein the antioxidant provitamin is selected from an alkaline metal salt and an alkaline earth metal salt.
4. The composition for enhancing the immunological effects as described in the above item 3, wherein the antioxidant provitamin is selected from a sodium salt, potassium salt, magnesium salt and calcium salt.
5. The composition for enhancing the immunological effects as described in any one of the above items 1 to 4, wherein the immunological effects of a vaccine is increased to 2 times or more in terms of the serum antibody unit.
6. The composition for enhancing the immunological effects as described in the above item 5, wherein the serum antibody unit is an antibody unit determined by hemagglutination inhibition (HI) reaction or passive hemagglutination (PHA) reaction.
7. The composition for enhancing the immunological effects as described in any one of the above items 1 to 4, wherein enhancing the immunological effects includes enhancing macrophage activity.
8. The composition for enhancing the immunological effects as described in any one of the above items 1 to 4, wherein enhancing the immunological effects includes enhancing induction of secretory IgA in the respiratory tract.
9. The composition for enhancing the immunological effects as described in any one of the above items 1 to 8, which is perorally administered.
10. The composition for enhancing the immunological effects as described in any one of the above items 1 to 8, which is administered intramuscularly, intradermally, intraveneously, subcutaneously, intranasally or externally.
11. The composition for enhancing the immunological effects as described in any one of the above items 1 to 10, which is used for mammals including human, birds or fishes.
12. The composition for enhancing the immunological effects as described in the above item 11, which is used for at least one animal under high-density feeding, selected from the group consisting of cattle, pig, fowl, horse, poultry, pet, ornamental animal, breeding marine animal and laboratory animal.
13. A method for enhancing the immunological effects of a vaccine for viruses, bacteria and/or infectious disease pathogens, which uses the composition for enhancing the immunological effects described in any one of the above items 1 to 12.
14. Feed, premix or drink comprising the composition for enhancing the immunological effects described in any one of the above items 1 to 12.
15. A method for using a composition for enhancing the immunological effects, comprising previously mixing the composition for enhancing the immunological effects described in any one of the above items 1 to 12 above with a vaccine and then administering the vaccine.

### DETAILED DESCRIPTION OF THE INVENTION

The present invention will be hereinafter described in detail.

By taking account of the fact that the stable activity-type provitamin exhibits excellent immunopotentiative activity in the host when injection administered into a body and also that L-ascorbic acid phosphates as one of the provitamin concerned exhibit antiviral activity based on the immunopotentiative activity against virus diseases, the present inventors have considered that the provitamin concerned must be effective in enhancing the phylactic effect of a vaccine based on the sthenia of circulating antibody or cell-mediated immunity, and have made investigations to use a vaccine and the provitamin concerned in combination.

Other than provitamins, some substances exhibit the above-described immunopotentiative activity. For example, a natural substance contained in the fruit body of Grifola erondosa exhibits, when perorally administered, immunopotentiative activity (see, International Journal of Immunopharmacology, Vol. 12, No. 6, 675-684 (1990)) and also fluctuates, when perorally administered, the lymphocyte subset in blood (see, Syokaki to Meneki (Digestive Apparatus and Immunity), No. 20, pp. 78-82 (1988)). As such, the effect by the combination of an immunopotentiator perorally administered and a vaccine is already known. Also, an agent for enhancing the immunological effect of a vaccine for viruses and bacterial, comprising a glucan having a β-1,3-glycoside bond as a main chain and originated from a mushroom was disclosed (see, JP-A-6-172217). However, the effect by the combination of a provitamin derivative and a vaccine is not yet known at present.

As a result of investigations by the present inventors, it is recognized that when a vaccine and the provitamin concerned are administered in combination, the circulating antibody and the production of the IgA antibody both are enhanced as compared with the administration of a vaccine alone, and also the macrophage activity as one index for the cell-mediated immunity is enhanced. Furthermore, it is verified that even when the amount of a vaccine used is greatly reduced, the phylactic effect obtained is sufficiently high. By these, the above-described problems all can be overcome.

The term "provitamin" as used in the present invention means a substance which is physically stable and inactive but under the action of an intercellular enzyme of the objective animal, expresses satisfactory vitamin activity.

Specifically, L-ascorbic acid derivatives and tocopheryl phosphates can be used as the provitamin of the present invention, however, among L-ascorbic acid derivatives, L-ascorbic acid-2-sulfate and the like are known to fail in exerting satisfactory activity in organisms because the organism does not have a hydrolase therefor, and therefore, these cannot be used in the present invention.

The L-ascorbic acid derivatives or the salts thereof for use in the present invention are represented by the following formula (1): 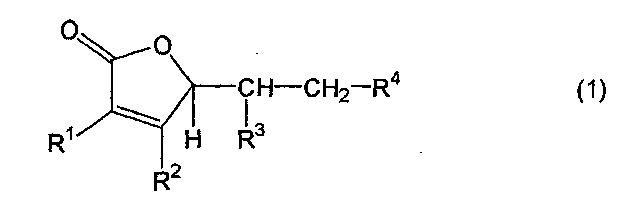 wherein R¹, R², R³ and R⁴ independently represent a hydroxyl group; an ester group of such a hydroxyl group with an inorganic acid or an organic acid; a glycoside group of such a hydroxyl group with a sugar; a ketal group of such a hydroxyl group with a ketone; or an acetal group of such a hydroxyl group with an aldehyde provided that R¹ and R² are not a hydroxyl group at the same time.

Specific examples of preferred L-ascorbic acid derivatives or the salts thereof include salts of L-ascorbic acid-2-phosphate or salts thereof, L-ascorbic acid-2-glycoside, 6-alkylcarbonyloxy-L-ascorbic acid-2-phosphate or salts thereof, etc. R⁴ is -OC(O)R in 6-alkylcarbonyloxy-L-ascorbic acid-2-phosphate or salts thereof, wherein R is preferably an alkyl group having 9 to 21 carbon atoms.

More specifically, 6-dodecanoyloxy-L-ascorbic acid-2-phosphate, 6-tetradecanoyloxy-L-ascorbic acid-2-phosphate, 6-palmytoyloxy-L-ascorbic acid-2-phosphate, 6-stearyloxy-L-ascorbic acid-2-phosphate, 6-(cis-9-octadecenoyloxy)-L-ascorbic acid-2-phosphate, 6-linoloxy-L-ascorbic acid-2-phosphate, 6-linoloxy-L-ascorbic acid-2-phosphate, 6-linolenyloxy-L-ascorbic acid-2-phosphate, 6-arachidonyloxy-L-ascorbic acid-2-phosphate, 5,6-O-benzylidene-L-ascorbic acid-2-phosphate or salts thereof. Monophosphate is a preferred 2-phosphate.

Metallic salts of L-ascorbic acid-2-phosphate for use in the present invention can be prepared by conventional methods.

As for L-ascorbic acid-2-glycoside, preferred are glucose glycoside such as L-ascorbic acid-2-glucoside ( 2-O-α-D-glucopyranosyl-L-ascorbic acid) and the like.

Examples of the salts of ascorbic acid derivatives include those with a cation such as ammonium, sodium, potassium, magnesium, calcium, strontium, barium, aluminum, iron, zinc, bismuth and organic amines. That is, the salt may be a salt with at least one of these cations. The ascorbic acid derivative and the salt thereof may be also a water adduct or hydrorate thereof.

Among the ascorbic acid derivatives and salts thereof usable in the present invention, those having an excellent effect include sodium salt, potassium salt and zinc salt of L-ascorbic acid-2-monophosphoric acid and L-ascorbic acid-2-glucoside. Particularly, sodium salt of L-ascorbic acid-2-monophosporic acid is preferred. Magnesium and calcium salts of L-ascorbic acid-2-monophosporic acid also have some effect, which, however, is inferior to that of the sodium salt.

The tocopherol in the tocopheryl phosphate and salts thereof for use in the present invention is represented by the following formula (2): 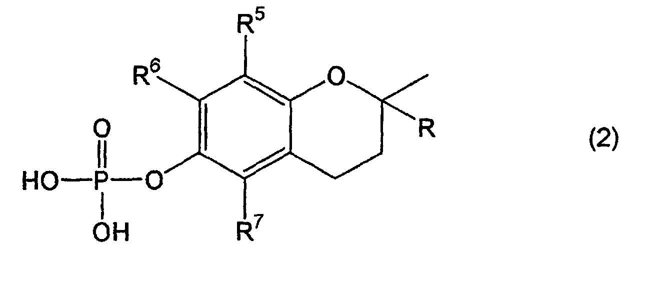 wherein R⁵, R⁶ and R⁷ represent hydrogen atom or methyl group, and R represents a long chain alkyl group:

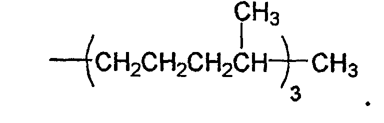

According to the substituents R⁵, R⁶ and R⁷, the tocopherol represented by the formula (2) is known as α-tocopherol (R⁵, R⁶ and R⁷: CH₃), β-tocopherol (R⁵ and R⁷: CH₃, R⁶: H), γ-tocopherol (R⁶ and R⁷: CH₃, R⁵: H), δ-tocopherol (R⁷: CH₃, R⁵ and R⁶: H), ζ2-tocopherol (R⁵ and R⁶: CH₃, R⁷: H) or η-tocopherol (R⁵ and R⁷: CH₃, R⁶: H). Among these, α-tocopherol is particularly preferred.

In addition, ζ1-tocopherol and ε-tocopherol where the long chain alkyl group represented by R which bonds to the carbon atom adjacent to the O atom in the benzopyrane structure of α-tocopherol or β-tocopherol is displaced by the following formula (3): 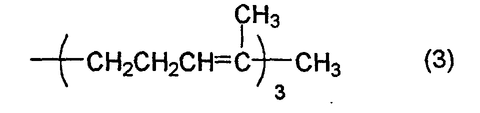 are known and either one can be a constituent element of the tocopheryl phosphate and salts thereof for use in the present invention.

Metallic salts of tocopheryl phosphate for use in the present invention can be prepared by conventional methods.

Examples of the salt of tocopheryl phosphate, which can be used in the present invention, include alkali metals and alkaline earth metals such as sodium, potassium, calcium, magnesium, aluminum, zinc and lithium. Among these, sodium salt is preferred because the solubility in water is high and the toxicity is low.

In order to effectively exert the immunopotentiative activity, the stable activity-type provitamin concerned is preferably administered by mixing it with feed or drinking water or by a syringe. If the case is so, the IgA antibody secreted on the respiratory airway or digestive tract mucosa shows no sthenia of production and therefore, the immunopotentiation of a influenza vaccine is not so much enhanced because the IgA antibody plays an important role in the phylaxis.

In the case where the provitamin concerned is perorally administered, it is recognized that the provitamin concerned appropriately contacts with the respiratory airway or digestive tract mucosa to stimulate the mucosa and thereby promote the secretion of the IgA antibody and by virtue of these three mechanisms of circulating antibody, cell-mediated immunity and IgA antibody, the living body is prevented from the virus infection. This effective immunopotentiative effect is estimated to occur also, for example, on the influenza vaccine. In fact, the present inventors have confirmed that when the provitamin is perorally administered, the IgA antibody shows significant enhancement of the production accompanying the administration of a Sendai virus vaccine closely related to the influenza virus and the phylactic effect is enhanced.

In the present invention, the activity of enhancing the immunological effects of a vaccine by the provitamin concerned is considered to result because the provitamin concerned non-specifically activates the prophylactic mechanism of a host. The provitamin concerned is found to have capability of accelerating the immunological effects of any kind of vaccines and to be widely applicable also to bacteria vaccines irrespective of the kind of the virus vaccine. Furthermore, this effectiveness is expected to extend even to protozoan diseases such as malaria.

In the composition of the present invention, the immunological effects of a vaccine is as high as 2 times or more, preferably 4 times or over in terms of the serum antibody unit. An immunological effects of less than 2 times the normal serum antibody unit is disadvantageously insufficient as the immunological effects of the composition of the present invention.

The provitamin concerned containing a stable activity-type provitamin may be administered into a body by injection administration, peroral administration or the like. The administration method may be appropriately selected according to the kind of vaccines, the condition of the host and the like. However, in view of the accelerated production of IgA antibody and the safety and easiness of administration, the peroral administration is preferred than the injection administration. The provitamin concerned may be previously mixed with a vaccine and then perorally administered.

The peroral administration is further advantageous in that the provitamin concerned needs not be purified to a high purity and for example, the bacteria which produce the provitamin concerned can be used in the form of a cell as it is or as a mixture thereof, so that the production cost can be reduced.

The composition of the present invention can be in the form of a solid composition and a liquid composition for peroral administration or a liguid composition for parental administration (parental injection).

Examples of the solid composition for peroral administration include tablet, capsule, powder medicine, granule or the like.

Capsules include a hard capsule and s soft capsule.

Such a solid composition comprises one or more active ingredients admixed with at least one inert diluent such as lactose, mannitol, mannit, glucose, hydroxypropylcellose, hydroxypropylcellose phthalate, microcrystalline cellulose, starch, polyvinylpyrrolidone, magnesium aluminometasilicate.

The composition can also comprise additives other than diluent in accordance with conventional methods, for examples, lubricants such as magnesium stearate, disintegrating agents such calcium carboxymethylcellulose, solubilizer and the like. Tablets may be optionally covered with a film of white sugar, gellatine, hydroxypropylcellose, hydroxypropylcellose phthalate and the like. Furthermore, capsules of digestible substances such as gellatine can be also used.

The liquid compositions for peroral administration include emulsion and solution, etc. Such a liquid composition comprises one or more active ingredients in a commonly used inert diluent such as purified water and ethanol. The compositions can also comprise additives such as humectants and elusifiers, sweetners, flavors, aroma and anticeptics.

The liquid compositions for parental injection include sterile aqueous and non-aqueous solvents, suspensions and emulsions. Examples of aqueous solvents and suspensions can be form of distilled water for injection and physiological saline. Examples of non-aqueous solvents and suspensions can be form of propylene glycol, polyethylene glycol, vegetable oils such as olive oils, and alcohols such as ethanol and the like.

The composition may also comprise other additives such as antiseptics, humectants, emulsifiers, dispersants, stabilizers, solubility aids (e.g. glutamic acid, aspartic acid). These additives may be sterilized by way of filtering with a bacteria-trapping filter, addition of a germicide or radiation. They can be formed as a sterilized solid composition and solved in a sterilized or aseptic distilled water for injection or the like solvent prior to use.

In the present invention, the effective dose of the provitamin which is an active ingredient is from 0.5 to 1,000 mg/kg per day for the peroral administration, and from 0.05 to 150 mg/kg for the injection administration. Preferably, the dose is from 5 to 500 mg/kg for the peroral administration and from 0.5 to 120 mg/kg for the injection administration, and more preferably from 10 to 100 mg/kg for the peroral administration and from 1 to 100 mg/kg for the injection administration.

Needless to say, the dose may vary depending on the various conditions, and therefore, the dose less than the above mentioned value may be suffice and the dose more than the above mentioned value may be required.

A vaccine is inoculated in a usual amount within a range of assuring the biological safety and the effect. Usually, an attenuated virus strain is used and this strain may be administered by a method commonly used in the vaccine administration and may be inoculated perorally or using a nebulizer or syringe. Depending on the kind of viruses, the amount inoculated varies but usually, a vaccine is suitably inoculated within the range from 10 to 10,000 CIU (cell infectious unit). With respect to the administration method, any method commonly used for the vaccine inoculation may be used and examples thereof include contacting with any mucosal tissue, such as oral, nasal or branchial mucosa, administration by nebulization, and injection such as intradermal injection.

The composition of the present invention can be blended with feed, premix or drink according to the above-described dose.

The composition for enhancing the vaccine effect of the present invention may be fed to an animal by blending it with a general feed such as a protein feed, carbohydrate feed, a feed additive or a feed ingredient. The optimal feed amount varies depending on the kind of animals, stage or eucrasia and therefore, may be appropriately determined according to the conditions where the composition is applied.

The composition for enhancing immunological effects of a vaccine of the present invention is effective for mammals including human, birds and fishes and particularly effective for animals under high-density feeding such as cattle, pig, fowl, horse, poultry, pet, breeding marine animal and laboratory animal. In general, the feeding environment of the animal under high-density feeding is restricted and therefore, the animal suffers from stress to readily cause deterioration of the immunological function and is prone to viral infection. Thus, it is revealed that the composition of the present invention has an effect of preventing viral infection which gives rise to health deterioration of a useful animal, and ameliorating the eucrasia.

### BEST MODE OF THE INVENTION

The present invention is described in greater detail below by referring to the Examples, however, the present invention is not limited to these Examples.

Hereinafter, L-ascorbic acid-2-monophosphate is referred to as "A2P" and α-tocopheryl phosphate is referred to as "VEP". For example, A2P-Mg indicates a magnesium salt of L-ascorbic acid-2-monophosphate and dl-VEP-Na indicates an Na salt of dl-α-tocopheryl phosphate.

### Example 1: Effect of provitamins on the circulating antibody unit (HIU) after the vaccine administration

The provitamins prepared to have a composition in the following Formulation Examples 1 to 4 were individually or in combination (hereinafter referred to as "provitamin") dissolved in a phosphate buffered saline (PBS) to a concentration of 0.5% and then continuously administered perorally or peritoneally to a mouse (ICR, 3 weeks old, male) from two weeks before the administration of a vaccine. The sample was administered such that APM per day was 80mg/kg-body weight in the case of peroral administration and the dose per day was 40 mg/kg-body weight in the case of injection.

The vaccine used here was TR-5 strain which is an attenuated strain of Sendai virus, and 500 CIU (cell infectious unit) thereof was nasally administered to a mouse under ether anesthesia. That is, a stock solution (10⁶ CIU/ml) of TR-5 strain was diluted with PBS and 50 µl of each sample was nasally administered. After the administration of vaccine, the serum was sampled in aging (inoculation day, after 7 days, after 14 days and after 21 days) and the antiviral antibody unit in the serum was measured by the hemagglutination inhibition (HI) reaction which inhibits the hemagglutination (HA) reaction.

The HA reaction is a reaction where a virus particle or an HA antigen adsorbs to an erythrocyte and combines the erythrocytes with each other to cause hemagglutination. In the measurement of the HA unit, 2-fold step dilution series of a virus solution are prepared, a constant amount of erythrocytes is added to each dilution and after the passing of a predetermined time, the agglutination image is examined. The HA unit (HAU) is the reciprocal of the highest dilution number in a test tube showing agglutination positive. The HA reaction is inhibited by previously adding an antiviral antibody to the virus solution. This is the HI reaction.

The HI antibody unit (HIU) is the reciprocal of the highest dilution number of a serum which completely inhibits the HA reaction attributable to 4 HAU virus antigen.

**Table 1**

| Composition(%) | | | | |
|---|---|---|---|---|
| | Formulation Example 1 | Formulation Example 2 | Formulation Example 3 | Formulation Example 4 |
| A2P-Mg | 100% | | 40% | 30% |
| A2P-Na | | 100% | 30% | 10% |
| A2P-Ca | | | | 10% |
| A2P-K | | | | 10% |
| dl-VEP-Mg | | | | 10% |
| dl-VEP-Na | | | | 10% |
| dl-VEP-Ca | | | 30% | 10% |
| dl-VEP-K | | | | 10% |

### Test Method:

To 25 µl of a serum subjected to 2-fold step dilution, 25 µl of a virus solution adjusted to 16 HAU was added and allowed to stand at room temperatures for 1 hour. Subsequently, 50 µl of a 0.5% chicken erythrocyte suspension was added and allowed to stand at 4°C for 1 hour. Thereafter, the HI unit was determined. The results are shown in Table 2.

**Table 2:**

| HIU | (Peroral Administration) | | | | (Peritoneal Administration) | | | |
|---|---|---|---|---|---|---|---|---|
| Days Passed After Infection | 0 | 7 | 14 | 21 | 0 | 7 | 14 | 21 |
| Vaccine-free segment | <16 | <16 | <16 | <16 | <16 | <16 | <16 | <16 |
| 500 CIU vaccine | <16 | <16 | <16 | 16 | <16 | <16 | 16 | 32 |
| 500 CIU vaccine + Formulation Example 1 | <16 | <16 | 16 | 64 | <16 | 16 | 32 | 64 |
| 500 CIU vaccine + Formulation Example 2 | <16 | <16 | 16 | 64 | <16 | <16 | 32 | 64 |
| 500 CIU vaccine + Formulation Example 3 | <16 | <16 | 64 | 64 | | | | |
| 500 CIU vaccine + Formulation Example 4 | <16 | 16 | 64 | 64 | | | | |

By using a vaccine and a provitamin, the circulating antibody unit was significantly increased as compared with the case of using the vaccine alone.

### Example 2: Effect of provitamin and vaccine on the activity of the macrophage

The administraion of provitamin and inoculation of a vaccine (500 CIU) were performed in the same manner as in Example 1. 14 Days after the inoculation of the vaccine, macrophages were sampled from the abdominal cavity of the mouse and the number of macrophages was counted through a microscope. Also, the activity of the macrophage was examined as follows. Fibrosarcoma SMT-5 (target cell) and the sampled macrophages (effector cell) were mixed at a ratio of 1:7 and cultured in CO₂ medium for 24 hours. 8 Hours before the completion of culture, ³H-thymidine was added and the amount of ³H-thymidine taken up into the residual target cells was measured by a liquid scintillation counter. From the value obtained, the activity of macrophage was determined. The amount of ³H-thymidine in the control group was the amount of ³H-thymidine taken up into the target cells where the macrophages were not added.

The results are shown in Table 3.

**Table 3:**

| Sample | Administration Route | Macrophage/ Body (×10⁶) | Macrophage Activity |
|---|---|---|---|
| 500 CIU vaccine | Peritoneal | 3.9 | 9.5 |
| 500 CIU vaccine + Formulation Example 1 | peritoneal | 9.1 | 22.1 |
| 500 CIU vaccine + Formulation Example 1 | Peroral | 6.8 | 20.5 |
| 500 CIU vaccine + Formulation Example 2 | Peroral | 6.5 | 20.2 |
| 500 CIU vaccine + Formulation Example 3 | Peroral | 6.3 | 21.3 |
| 500 CIU vaccine + Formulation Example 4 | Peroral | 7.1 | 23.7 |

By using a vaccine and a provitamin in combination, the production of macrophage was enhanced and the activation was revealed, as compared with the case of using the vaccine alone.

### Example 3: Effect of rovitamin on secretory IgA induction by Sendai virus live vaccine

The administraion of provitamin and inoculation of a vaccine were performed in the same manner as in Example 1. 14 Days after the inoculation of vaccine, the mouse was sacrificed, the trachea and bronchia were twice washed with 1 ml of PBS (phosphate buffered saline), and IgA recovered in PBS was measured by the enzyme linked immunosorbent assay (ELISA).

### ELISA:

Sendai virus was fixed on a plate and reacted with a test solution. Thereafter, peroxidase marker anti-mouse IgA immunoglobulin was reacted and then phenylenediamine 2HCl was added to cause coloration. OD 492 was measured and from the calibration curve obtained based on the standard IgA, the IgA amount in the test solution was determined.

The results are shown together in Table 4.

**Table 4:**

| Sample | Administration Route | IgA (U/µg) |
|---|---|---|
| Control group (not treated) | | 14 |
| 500 CIU vaccine | Peritoneal | 19 |
| 500 CIU vaccine + Formulation Example 1 | Peritoneal | 69 |
| 500 CIU vaccine + Formulation Example 1 | Peroral | 51 |
| 500 CIU vaccine + Formulation Example 2 | Peroral | 69 |
| 500 CIU vaccine + Formulation Example 3 | Peroral | 68 |
| 500 CIU vaccine + Formulation Example 4 | Peroral | 70 |

By using a vaccine and a provitamin in combination, the induction of secretory IgA was enhanced, as compared with the case of using the vaccine alone.

### Example 4

The administraion of provitamin and inoculation of a vaccine were performed in the same manner as in Example 1, and 14 days after the inoculation of attenuated vaccine, Sendai virus strongly toxic strain (1.7×10⁷ CIU/ml) was subjected to 15-hold dilution in PBS. Subsequently, 70 µl of the dilution (about 8×10⁴ CIU: 15 LD₅₀) was nasally infected to a mouse under ether anesthesia and 20 days after the infection with the strongly toxic strain, the survival rate of mice was examined. The results are shown in Table 5.

### (Measurement of LD₅₀)

The test solution was step-diluted and each dilution was used for a group of animals. The life and death were examined and the point of dilution degree where 50% of death was recognized was designated as LD₅₀.

**Table 5**

| | | Survival Rate (number of survival mice/number of mice tested) | | |
|---|---|---|---|---|
| | | Amount of Vaccine Inoculated (CIU) | | |
| Sample | Administration Route | 0 | 50 | 500 |
| Control group (PBS administration) | Peritoneal | 0/10 | 0/10 | 2/10 |
| Control group (PBS administration) | Peroral | 0/10 | 0/10 | 1/10 |
| Formulation Example 1 | Peritoneal | 0/10 | 8/10 | 8/10 |
| Formulation Example 1 | Peroral | 0/10 | 9/10 | 9/10 |
| Formulation Example 2 | Peroral | 1/10 | 9/10 | 10/10 |
| Formulation Example 3 | Peroral | 1/10 | 8/10 | 10/10 |

By using a vaccine and a provitamin, the survival rate was increased as compared with the case of using the vaccine alone.

### INDUSTRIAL APPLICABILITY

According to the composition for enhancing immunological effects of the present invention which contains at least one compound selected from L-ascorbic acid derivatives and α-tocopheryl phosphates, the provitamins are resistant to the decomposition by a digestive enzyme *in vivo*, very low in the toxicity, almost free of an adverse reaction even by the injection administration. Accordingly, the composition is, as prominent characteristic features, completely nulled in the toxicity in the case of peroral administration. In addition, the provitamin of the present invention is converted into a natural nutrient and since the toxicity is low, the provitamin may be taken as food or animal feed and even in this case, a sufficiently high vaccine enhancing effect can be expected. In the case of using the provitamin concerned as food or feed, purification to a high purity is not necessary but the coarse product or a culture dry product in the provitamin production can provide a sufficiently high expected effect as it is and therefore, the composition for enhancing the immunological effects of a vaccine for viruses, bacteria and/or infectious disease pathogens is useful over a wide range.

## Claims

1. A composition for enhancing the immunological effects of a vaccine for viruses, bacteria and/or infectious disease pathogens, comprising a stable activity-type antioxidant provitamin.

2. The composition for enhancing the immunological effects as claimed in claim 1, wherein the antioxidant provitamin is at least one compound selected from L-ascorbic acid derivatives, α-tocopheryl phosphates and salts thereof.

3. The composition for enhancing the immunological effects as claimed in claim 1 or 2, wherein the antioxidant provitamin is selected from an alkaline metal salt and an alkaline earth metal salt.

4. The composition for enhancing the immunological effects as claimed in claim 3, wherein the antioxidant provitamin is selected from a sodium salt, potassium salt, magnesium salt and calcium salt

5. The composition for enhancing the immunological effects as claimed in any one of claims 1 to 4, wherein the immunological effects of a vaccine is increased to 2 times or more in terms of the serum antibody unit.

6. The composition for enhancing the immunological effects as claimed in claim 5, wherein the serum antibody unit is an antibody unit determined by hemagglutination inhibition (HI) reaction or passive hemagglutination (PHA) reaction.

7. The composition for enhancing the immunological effects as claimed in any one of claims 1 to 4, wherein enhancing the immunological effects includes enhancing macrophage activity.

8. The composition for enhancing the immunological effects as claimed in any one of claims 1 to 4, wherein enhancing the immunological effects includes enhancing induction of secretory IgA in the respiratory tract.

9. The composition for enhancing the immunological effects as claimed in any one of claims 1 to 8, which is perorally administered.

10. The composition for enhancing the immunological effects as claimed in any one of claims 1 to 8, which is administered intramuscularly, intradermally, intraveneously, subcutaneously, intranasally or externally.

11. The composition for enhancing the immunological effects as claimed in any one of claims 1 to 10, which is used for mammals including human, birds or fishes.

12. The composition for enhancing the immunological effects as claimed in claim 11, which is used for at least one animal under high-density feeding, selected from from the group consisting of cattle, pig, fowl, horse, poultry, pet, ornamental animal, breeding marine animal and laboratory animal.

13. A method for enhancing the immunological effects of a vaccine for viruses, bacteria and/or infectious disease pathogens, which uses the composition for enhancing the immunological effects described in any one of claims 1 to 12.

14. Feed, premix or drink comprising the composition for enhancing the immunological effects described in any one of claims 1 to 12.

15. A method for using a composition for enhancing the immunological effects, comprising previously mixing the composition for enhancing the immunological effects described in any one of claims 1 to 12 with a vaccine and then administering the vaccine.
